# EUROPEAN PATENT SPECIFICATION

(11) **EP 3 731 812 B1**
(45) Date of publication and mention of the grant of the patent: **26.11.2025**
(21) Application number: 18899014.7
(22) Date of filing: 28.12.2018
(51) Int. Cl.: A61K 9/00, A61K 9/20, A61K 31/4196

(54) **WATER-DISPERSIBLE TABLET FORMULATIONS COMPRISING DEFERASIROX**
WASSERDISPERGIERBARE TABLETTENFORMULIERUNG MIT DEFERASIROX
FORMULATIONS DE COMPRIMÉS DISPERSIBLES DANS L'EAU COMPRENANT DU DÉFÉRASIROX

(30) Priority: 29.12.2017 TR 201722910
(43) Date of publication of application: 04.11.2020
(73) Proprietor: Sanovel Ilac Sanayi ve Ticaret A.S., Sisli - Istanbul (TR)
(72) Inventor: TÜRKYILMAZ, Ali, 34460 Istanbul (TR); MURATOGLU, Yesim, 34460 Istanbul (TR); YILDIRIM, Ediz, 34398 Istanbul (TR)
(74) Representative: Genç Ilhan, Oznur
(86) International application number: PCT/TR2018/050931
(87) International publication number: WO 2019/151967

(56) References cited:
- EP-A1- 2 062 572
- EP-A1- 2 946 771
- WO-A1-2004/035026
- WO-A1-2014/067501
- WO-A1-2016/167729
- WO-A1-2018/208242

## Description

### Field of Invention

The present invention relates to formulations comprising deferasirox or a pharmaceutically acceptable salt thereof. More particularly, the present invention relates to water-dispersible tablet formulations of deferasirox.

### Background of Invention

Deferasirox is an iron-binding agent used for the treatment of iron overload. Its chemical name is 4-[3,5-bis(2-hydroxyphenyl)[1,2,4]triazole-1-yl]benzoic acid and has the structure shown below in formula I.

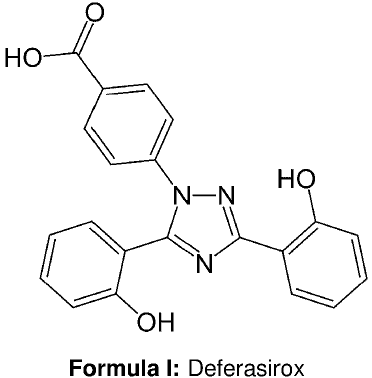

Deferasirox is indicated in the treatment of iron overload for reducing iron-related morbidity and mortality in transfusion-dependent anemia, particularly in mild and severe thalassemia, and in sickle cell anemia. At the same time, deferasirox can be used in the treatment of hemochromatosis. The red blood cells in the body of patients afflicted with mild and severe thalassemia, sickle-cell anemia, and hemochromatosis are exposed to a very rapid destruction and a significant amount of blood transfusion is given to the patients in the need thereof. If the excessive amount of iron following a blood transfusion is not eliminated from the body, it may cause organ failures. Deferasirox is an effective active agent in eliminating excessive iron from the body.

Deferasirox is the first oral drug approved for the treatment of iron overload in the United States. It was approved by FDA (Food and Drug Administration) in November 2005.

Deferasirox, which is commercially-available in water-dispersible forms (EXJADE^{®}) was first disclosed in the patent WO97/49395, wherein it was indicated for the treatment of diseases caused by excessive iron in the body.

Various water-dispersible tablet formulations comprising deferasirox can be found in the prior art.

The patent application EP2946771 A1 of Sanovel Ilac Sanayi ve Ticaret A.S. discloses a water-dispersible tablet comprising deferasirox and colloidal silicon dioxide, characterized in that 50.0% by weight of the total amount of colloidal silicon dioxide is provided in the granular phase, and 50.0% by weight of the total amount of colloidal silicon dioxide is provided in the powder phase.

The patent application EP1734924A1 of Novartis, for instance, discloses a dispersible tablet comprising deferasirox in an amount of 42% to 65% by weight of the total tablet.

The patent application EP1940360 of Novartis, in turn, discloses a dispersible tablet formulation comprising deferasirox in an amount of 42% to 65% by weight based on the total weight of the tablet, and at least one excipient.

The patent TR2011/03691 of the firm Ali Raif discloses a water-dispersible tablet formulation comprising deferasirox in an amount above 40,5% and below 41,55% of the total weight of the tablet.

The patent TR2009/05371 of Novartis discloses a dispersible tablet formulation comprising deferasirox in an amount of 5% to 40% by weight based on the total weight of the tablet and a disintegrant in an amount of 10% to 35% by weight based on the total weight of the tablet. That document further discloses a complex production method consisting of 4 steps. The patents and patent applications according to the prior art referred to above disclose similar production methods. Therefore, a simple, time-saving and cost-efficient production method would be desirable.

125 mg, 250 mg and 500 mg tablet formulations of deferasirox are available, but particularly since the total tablet weight of the 500 mg dose is too high, it is not suitable for use as oral tablets. It is known that administering oral tablets with a large tablet size is problematic in patients with difficulty swallowing. The water-dispersible tablet formulations comprising deferasirox have been developed for this reason.

The term "water-dispersible tablet" refers to a tablet, which is administered after it is dissolved in water.

It is known that deferasirox is poorly soluble in water. For this reason, the most-frequently encountered difficulty in producing water-dispersible tablets comprising deferasirox is the solubility thereof.

Additionally, the particles obtained in the production of dispersible tablets of deferasirox have low flow properties and are prone to adhere to each other because of the low density and the electrostatic characteristics of deferasirox.

In order to fulfill all the requirements described above, a special drug formulation should be adapted particularly by a careful selection of the excipients to be used. Therefore, water-dispersible tablets comprising deferasirox are the desired, which would not lead to losses in the active agents or excipients during production, i.e. would have a high production yield, would rapidly dissolve in water, have good flow properties, and would be simply compressed into tablets. Other advantages and embodiments of the present invention will be clarified in the following description.

### Detailed Description of Invention

The main object of the present invention is to provide a water-dispersible tablet formulation comprising deferasirox or a pharmaceutically acceptable salt thereof which overcomes the problems referred to above using suitable excipients in the specific amounts.

A further object of the present invention is to provide a water-dispersible tablet formulation comprising a pharmaceutically effective amount of deferasirox and having a desired level of solubility and dissolution rate.

A further object of the present invention is to provide a water-dispersible tablet formulation of deferasirox or a pharmaceutically acceptable salt thereof, which has suitable mechanical strength (e.g. adequate hardness and low erosion) to be effectively processed in high-speed tablet presses and transported in low-cost packages.

The water-dispersible tablet formulation of the present invention comprises deferasirox or a pharmaceutically acceptable salt thereof and crospovidone as a disintegrant, wherein crospovidone is in an amount of between 37.00 and 38.00% by weight of the total formulation, wherein the formulation further comprises a surfactant which is sodium lauryl sulfate and a binder which is copovidone, wherein the weight ratio of sodium lauryl sulfate to copovidone is between 0.10 and 3.50.

It has been surprisingly found that, a water-dispersible tablet formulation of deferasirox or a pharmaceutically acceptable salt thereof with a good dissolution and solubility is developed by using a disintegrant in the formulation in an amount of between 37.00 and 38.00% by weight of the total formulation.

Suitable disintegrants are selected from the group selected from the group comprising crospovidone, croscarmellose sodium, polyethylene oxide, sodium starch glycolate, microcrystalline cellulose, sodium carboxymethyl starch, soy polysaccharide, cross-linked alginic acid, copovidone, gellan gum, xanthan gum, calcium silicate or ion exchange resins or mixtures thereof.

The compositions of the claimed invention must comprise a disintegrant which is crospovidone.

During the development study of the invention, the inventors have found that, better dissolution results are obtained by choosing crospovidone as the disintegrant.

In one embodiment, deferasirox or a pharmaceutically acceptable salt thereof is present in the formulation in an amount of between 10.00% and 50.00%, preferably between 15.00% and 35.00%, more preferably between 20.00% and 30.00% by weight of the total formulation. In one embodiment, the water-dispersible tablet formulation further comprises at least one pharmaceutically acceptable excipient which is selected from a group comprising binders, surfactants, fillers, glidants, lubricants or mixtures thereof. Only compositions comprising a surfactant which is sodium lauryl sulfate and a binder which is copovidone are according to the claimed invention.

Suitable surfactants are selected from the group comprising sodium lauryl sulfate, cetylpyridinium chloride, docusate sodium, lauric acid, polyoxyethylene sorbitan fatty acid esters (polysorbate), phospholipids, cetrimide or mixtures thereof.

The compositions of the claimed invention must comprise a surfactant which is sodium lauryl sulfate.

Sodium lauryl sulfate is an anionic surfactant having a hydrophobic and a hydrophilic group.

Based on this structural property, it is a surfactant which is very effective in increasing the solubility of active agents such as deferasirox which do not dissolve in water. The hydrophobic groups of sodium lauryl sulfate surround the deferasirox molecule and prevent the contact of deferasirox molecules with water. Only the hydrophilic groups of sodium lauryl sulfate contact with water and makes deferasirox dissolve in water.

According to one embodiment, sodium lauryl sulfate is present in the formulation in an amount of between 0.40% and 3.50%, preferably between 0.40% and 2.50% by weight of the total formulation. It has been found that, when sodium lauryl sulfate is used in amounts of this range, the solubility of the formulation enhances.

Furthermore, the weight ratio of the crospovidone to sodium lauryl sulfate is more than 10.00, preferably more than 11.00. More preferably the weight ratio of the crospovidone to sodium lauryl sulfate is between 11.00 and 80.00. The weight ratio of the crospovidone to sodium lauryl sulfate is very important since more soluble tablets were obtained by setting the ratio as mentioned above. Thus, since the tablets obtained disintegrate easily in water, the patient compliance was also improved.

Suitable binders are selected from a group comprising copovidone, xanthan gum, guar gum, carbomer, pregelatinized starch, povidone, , carnauba wax, hydroxypropyl methyl cellulose, pullulan, polymethacrylate, glyceryl behenate, hydroxypropyl cellulose, carboxymethyl cellulose, methyl cellulose, hydroxyethyl cellulose, sodium carboxymethyl cellulose, carboxymethyl cellulose calcium, ethyl cellulose, microcrystalline cellulose, polymethacrylates, polyethylene oxide, polyvinyl alcohol, polycarbophil, polyvinyl acetate and its copolymers, gelatin, starch, alginate, carrageen, collagen, agar, pectin, hyaluronic acid, cellulose acetate phthalate, hydroxypropyl starch, hydroxyethyl methyl cellulose, polaxomer, polyethylene glycol, sugars, glucose syrups, natural gums, tragacanth gum, polyacrylamide, aluminum hydroxide, benthonite, laponite, cetostearyl alcohol, polyoxyethylene-alkyl ethers, acacia mucilage, polydextrose or mixtures thereof.

In one embodiment, the binder is present in the formulation in an amount of between 0.50% and 10.00%, preferably 1.00% and 5.00% by weight of the total formulation.

The compositions of the claimed invention must comprise a binder which is copovidone (polyvinylpyrrolidone-vinyl acetate copolymer).

According to a preferred embodiment, copovidone is copovidone fine.

Fine copovidone is the copovidone powder with the D50 value ranging between 1-180 µm, preferably lower than 60 µm.

A further object of the present invention is to provide highly-soluble water-dispersible tablets comprising deferasirox or a pharmaceutically acceptable salt thereof. Accordingly, it was surprisingly found that when the binder and the surfactant are used in specific amounts, the solubility of the formulation increases.

The weight ratio of sodium lauryl sulfate to copovidone is between 0.10 and 3.50. This specific range helps to enhance the solubility of the formulation.

Suitable fillers are selected from a group comprising lactose monohydrate, microcrystalline cellulose, sorbitol, sucrose, inorganic salts, calcium salts, dextrose, dicalcium phosphate, sodium chloride, dextrates, lactitol, maltodextrin, sucrose-maltodextrin mixtures, xylitol, trehalose, lactose, spray-dried lactose monohydrate or mixtures thereof.

In one preferred embodiment, the filler is lactose monohydrate, microcrystalline cellulose or mixtures thereof.

In one preferred embodiment, the formulation comprises lactose monohydrate and microcrystalline cellulose as fillers.

According to these embodiments the filler is present in the formulation in an amount of between 10.00% and 40.00%

Suitable lubricants are selected from a group comprising sodium stearyl fumarate, sodium lauryl sulfate, magnesium lauryl sulfate, magnesium stearate, fumaric acid, sodium chloride benzoate, sodium chloride acetate, glyceryl palmitostearate, hydrogenated natural oils, zinc stearate, calcium stearate, silica, talk, stearic acid, polyethylene glycol, paraffin or mixtures thereof.

In one preferred embodiment, the lubricant used is magnesium stearate.

According to this embodiment, the lubricant is present in the formulation in an amount of between 0.01% and 5.00%, preferably between 0.10% and 1.00% by weight of the total formulation.

Suitable glidants are selected from colloidal silicon dioxide, talc, aluminium silicate or mixtures thereof.

In one preferred embodiment, the glidant is colloidal silicon dioxide.

According to this embodiment, the glidant is present in the formulation in an amount of 0.01% and 5.00%, preferably between 0.10% and 1.00% by weight of the total formulation.

In one embodiment, the formulation comprises;
a. deferasirox or a pharmaceutically acceptable salt thereof 10.00-50.00%
b. crospovidone 37.00-38.00%
c. lactose monohydrate 5.00-15.00%
d. microcrystalline cellulose 5.00-25.00%
e. copovidone 0.50-10.00%
f. sodium lauryl sulfate 0.40-3.50%
g. colloidal silicon dioxide 0.01-5.00%
h. magnesium stearate 0.01-5.00%

According to all these embodiments, the below given formulations can be used in the pharmaceutical composition subjected to the invention. These examples are not limiting the scope of the present invention and should be considered under the light of the foregoing detailed disclosure.

### Example 1: Water-dispersible tablet comprising deferasirox

| **Ingredients** | **Amount** (%) |
|---|---|
| Deferasirox | 10.00-50.00 |
| Crospovidone | 37.00-38.00 |
| Lactose monohydrate | 5.00-15.00 |
| Microcrystalline cellulose | 5.00-25.00 |
| Copovidone | 0.50-10.00 |
| Sodium lauryl sulfate | 0.40-3.50 |
| Colloidal silicon dioxide | 0.01-5.00 |
| Magnesium stearate | 0.01-5.00 |

### Example 2: Water-dispersible tablet comprising deferasirox

| **Ingredients** | **Amount** (%) |
|---|---|
| Deferasirox | 20.00-30.00 |
| Crospovidone | 37.50 |
| Lactose monohydrate | 5.00-15.00 |
| Microcrystalline cellulose | 5.00-25.00 |
| Copovidone | 1.00-5.00 |
| Sodium lauryl sulfate | 0.40-3.50 |
| Colloidal silicon dioxide | 0.1-1.00 |
| Magnesium stearate | 0.1-1.00 |

The pharmaceutical compositions mentioned above are prepared by following these steps:
a. Mixing deferasirox or a pharmaceutically acceptable salt thereof, the half of crospovidone, lactose monohydrate and copovidone
b. Solving sodium lauryl sulfate in water to form a granulation agent
c. Granulating the mixture with the granulation agent
d. Sieving, drying and adding the other half of crospovidone to the mixture and mixing
e. Adding colloidal silicon dioxide to the mixture and mixing
f. Adding magnesium stearate to the mixture and mixing
g. Compressing the mixture to form tablets.

## Claims

1. A water-dispersible tablet formulation comprising deferasirox or a pharmaceutically acceptable salt thereof and crospovidone as a disintegrant, wherein crospovidone is in an amount of between 37.00 and 38.00% by weight of the total formulation, wherein the formulation further comprises a surfactant which is sodium lauryl sulfate and a binder which is copovidone, wherein the weight ratio of sodium lauryl sulfate to copovidone is between 0.10 and 3.50.

2. The water-dispersible tablet formulation according to claim 1, wherein said deferasirox or a pharmaceutically acceptable salt thereof is present in the formulation in an amount of between 10.00% and 50.00%, preferably between 15.00% and 35.00%, more preferably between 20.00% and 30.00% by weight of the total formulation.

3. The water-dispersible tablet formulation according to claims 1 or 2, wherein the formulation further comprising at least one pharmaceutically acceptable excipient which is selected from a group comprising fillers, glidants, lubricants or mixtures thereof.

4. The water-dispersible tablet formulation according to claim 3, wherein the surfactant is present in the formulation in an amount of between 0.40% and 3.50% by weight of the total formulation.

5. The water-dispersible tablet formulation according to any preceding claims, wherein the weight ratio of crospovidone to sodium lauryl sulfate is more than 10.00.

6. The water-dispersible tablet formulation according to claim 3, wherein the binder is present in the formulation in an amount of between 0.50% and 10.00%.

## Patentansprüche

1. Eine wasserdispergierbare Tablettenformulierung, die Deferasirox oder ein pharmazeutisch akzeptables Salz davon und Crospovidon als Sprengmittel umfasst, wobei Crospovidon in einer Menge zwischen 37,00 und 38,00 % des Gesamtgewichts der Formulierung vorliegt, wobei die Formulierung ferner ein Tensid, nämlich Natriumlaurylsulfat, und ein Bindemittel, nämlich Copovidon, umfasst, wobei das Gewichtsverhältnis von Natriumlaurylsulfat zu Copovidon zwischen 0,10 und 3,50 liegt.

2. Die wasserdispergierbare Tablettenformulierung gemäß Anspruch 1, wobei das Deferasirox oder ein pharmazeutisch verträgliches Salz davon in der Formulierung in einer Menge zwischen 10,00 % und 50,00 %, vorzugsweise zwischen 15,00 % und 35,00 %, noch bevorzugter zwischen 20,00 % und 30,00% des Gewichts der Gesamtformulierung vorhanden ist.

3. Die wasserdispergierbare Tablettenformulierung gemäß Anspruch 1 oder 2, wobei die Formulierung ferner mindestens einen pharmazeutisch akzeptablen Hilfsstoff umfasst, der aus einer Gruppe ausgewählt ist, die Füllstoffe, Gleitmittel, Schmiermittel oder Mischungen davon umfasst.

4. Die wasserdispergierbare Tablettenformulierung gemäß Anspruch 3, wobei das Tensid in der Formulierung in einer Menge zwischen 0,40 % und 3,50 % des Gewichts der Gesamtformulierung vorhanden ist.

5. Wasserdispergierbare Tablettenformulierung gemäß einem der vorstehenden Ansprüche, wobei das Gewichtsverhältnis von Crospovidon zu Natriumlaurylsulfat mehr als 10,00 beträgt.

6. Die wasserdispergierbare Tablettenformulierung gemäß Anspruch 3, wobei das Bindemittel in der Formulierung in einer Menge zwischen 0,50 % und 10,00 % vorhanden ist.

## Revendications

1. Formulation de comprimés dispersibles dans l'eau comprenant du déférasirox ou un sel pharmaceutiquement acceptable de celui-ci et de la crospovidone comme désintégrant, dans laquelle la crospovidone est présente en une quantité comprise entre 37,00 % et 38,00 % en poids de la formulation totale, la formulation comprenant en outre un tensioactif, qui est le laurylsulfate de sodium, et un liant, qui est la copovidone, le rapport pondéral du laurylsulfate de sodium à la copovidone étant compris entre 0,10 et 3,50.

2. Formulation de comprimés dispersibles dans l'eau selon la revendication 1, dans laquelle le déférasirox ou un sel pharmaceutiquement acceptable de celui-ci est présent dans la formulation en une quantité comprise entre 10,00 % et 50,00 %, de préférence entre 15,00 % et 35,00 %, plus préférablement entre 20,00 % et 30,00 % en poids de la formulation totale.

3. Formulation de comprimés dispersibles dans l'eau selon les revendications 1 ou 2, dans laquelle la formulation comprend en outre au moins un excipient pharmaceutiquement acceptable choisi parmi des agents de remplissage, des agents glissants, des lubrifiants ou des mélanges de ceux-ci.

4. Formulation de comprimés dispersibles dans l'eau selon la revendication 3, dans laquelle le tensioactif est présent dans la formulation en une quantité comprise entre 0,40 % et 3,50 % en poids de la formulation totale.

5. Formulation de comprimés dispersibles dans l'eau selon l'une quelconque des revendications précédentes, dans laquelle le rapport pondéral entre la crospovidone et le laurylsulfate de sodium est supérieur à 10,00.

6. Formulation de comprimés dispersibles dans l'eau selon la revendication 3, dans laquelle le liant est présent dans la formulation en une quantité comprise entre 0,50 % et 10,00 % en poids de la formulation totale.
